# EUROPEAN PATENT APPLICATION

(11) **EP 4 449 909 A1**
(43) Date of publication of application: **23.10.2024**
(21) Application number: 21968099.8
(22) Date of filing: 15.12.2021
(51) Int. Cl.: A24F 40/40, A24F 40/90

(54) **FLAVOR INHALER**

(71) Applicant: Japan Tobacco Inc., Tokyo 105-6927 (JP)
(72) Inventor: KAMADA, Ryuji, Tokyo 130-8603 (JP); ICHINOSE, Atsushi, Tokyo 130-8603 (JP)
(74) Representative: Hoffmann Eitle
(86) International application number: PCT/JP2021/046182
(87) International publication number: WO 2023/112182

(57) **Abstract**

A flavor inhaler configured to generate an aerosol by heating a smokable substance is provided. This flavor inhaler includes a first board including an electronic element, and a housing containing the first board. The first board includes a first surface and a second surface. The second surface is opposite from the first surface, and is located at a longer distance from the housing than a distance between the first surface and the housing. The flavor inhaler further includes a component facing the second surface of the first board, and a first heat dissipation member interposed between a portion of the second surface of the first board that corresponds to the electronic element and the component.

## Description

### TECHNICAL FIELD

The present invention relates to a flavor inhaler.

### BACKGROUND ART

Conventionally, there have been known flavor inhalers for inhaling a flavor or the like without burning a material. Such a flavor inhaler is equipped with a rechargeable battery for supplying power to a heater that heats a consumable (for example, refer to PTL 1).

### CITATION LIST

### PATENT LITERATURE

PTL 1: Japanese Unexamined Patent Application Publication (Translation of PCT Application) No. 2019-521656

### SUMMARY OF INVENTION

### TECHNICAL PROBLEM

Such a flavor inhaler is subjected to a demand for reducing the time taken to charge the battery. If a current of a higher value is supplied to the battery to reduce the time taken to charge the battery, a charging IC (Integrated Circuit) for controlling the charging generates heat to have a higher temperature than a conventionally reached temperature. This case leads to a local increase in the temperature of the charging IC mounted on a PCB (printed circuit board), thereby necessitating appropriate heat dissipation therefrom. The appropriate heat dissipation is necessary not only for the charging IC but also when an electronic element mounted on the PCB generates heat to have a high temperature. It is preferable to dissipate such heat to outside a housing of the flavor inhaler. Then, one conceivable measure therefor is to arrange a member for heat dissipation between the PCB and the housing so as to contact the PCB (the electronic element) and the housing. However, because the PCB, the heater, and the like are constructed in advance, and the flavor inhaler is assembled by placing them into the housing after that, it is not preferable to arrange the heat dissipation member so as to contact the PCB (the electronic element) and the housing in consideration of the assembling of the flavor inhaler.

One of objects of the present invention is to dissipate heat of an electronic element without hindering assembling of a flavor inhaler.

### SOLUTION TO PROBLEM

According to a first aspect, a flavor inhaler configured to generate an aerosol by heating a smokable substance is provided. This flavor inhaler includes a first board including an electronic element, and a housing containing the first board. The first board includes a first surface and a second surface. The second surface is opposite from the first surface, and is located at a longer distance from the housing than a distance between the first surface and the housing. The flavor inhaler further includes a component facing the second surface of the first board, and a first heat dissipation member interposed between a portion of the second surface of the first board that corresponds to the electronic element and the component.

According to the first aspect, heat of the electronic element of the first board can be transferred (dissipated) to the component facing the second surface of the first board via the first heat dissipation member, and therefore local heat generated in the first board can be transferred to the above-described component while being dispersed. In addition thereto, since the first heat dissipation member is interposed between the above-described component and the first board, the first heat dissipation member can be prevented from contacting the housing to hinder the assembling of the flavor inhaler when the first board, the first heat dissipation member, and the above-described component are placed into the housing.

According to a second aspect, in the first aspect, the electronic element is a charging IC for controlling charging of a battery.

According to the second aspect, the heat can be dissipated from the charging IC to the above-described component, and therefore an increase in the temperature of the charging IC can be suppressed even when a current of a higher value is supplied to the battery to reduce the time taken to charge the battery compared with a conventionally spent time.

According to a third aspect, in the first or second aspect, the first heat dissipation member is a heat dissipation pad containing silicone.

According to the third aspect, the heat can be efficiently dissipated from the first board to the above-described component via the first heat dissipation member due to the interposed arrangement of the first heat dissipation member between the first board and the above-described component.

According to a fourth aspect, in the third aspect, the first heat dissipation member is compressed by the second surface of the first board and the above-described component.

The heat dissipation pad containing silicone can efficiently transfer heat by being disposed in contact with a heat dissipation target so as to be compressed. According to the fourth aspect, the heat can be efficiently dissipated from the first board to the above-described component via the first heat dissipation member due to the compression of the first heat dissipation member between the first board and the above-described component.

According to a fifth aspect, in any of the first to fourth aspects, the component is a second board having a surface facing the second surface of the first board.

According to the fifth aspect, the local heat generated in the first board can be transferred to the surface of the second board while being dispersed, and therefore a local increase in the temperature of the second board can be suppressed.

According to a sixth aspect, in any of the first to fifth aspects, the electronic element is mounted on the first surface of the first board.

According to the sixth aspect, the heat is dispersed in an in-plane direction in the first board when the heat of the electronic element is transferred from the first surface to the second surface of the first board, and therefore the locally generated heat of the electronic element can be transferred to the first heat dissipation member while being dispersed by the first board.

According to a seventh aspect, in any of the first to sixth aspects, the flavor inhaler further includes a battery, and a second heat dissipation member interposed between a surface of the battery and an inner surface of the housing.

If a current of a higher value is supplied to the battery to reduce the time taken to charge the battery, the battery also generates heat to have a higher temperature than a conventionally reached temperature. According to the seventh aspect, the heat of the battery can be transferred (dissipated) to the housing via the second heat dissipation member.

According to an eighth aspect, in the seventh aspect, the housing includes a first housing including a bottom wall, a circumferential wall, and an opening defined by the circumferential wall, and a second housing configured to close the opening. The second heat dissipation member is interposed between the surface of the battery and an inner surface of the bottom wall.

According to the eighth aspect, the second heat dissipation member can be interposed between the surface of the battery and the inner surface of the bottom wall by placing the battery into the first housing via the opening. Therefore, the second heat dissipation member can be prevented from hindering the assembling of the flavor inhaler.

### BRIEF DESCRIPTION OF DRAWINGS

Fig. 1A is a schematic front view of a flavor inhaler according to an embodiment of the present invention.
Fig. 1B is a schematic top view of the flavor inhaler according to the present embodiment.
Fig. 1C is a schematic bottom view of the flavor inhaler according to the present embodiment.
Fig. 2 is a schematic side cross-sectional view of a consumable.
Fig. 3 is a schematic cross-sectional view of the flavor inhaler as viewed from arrows 3 and 3 illustrated in Fig. 1B.
Fig. 4 is a schematic cross-sectional view of the flavor inhaler as viewed from arrows 4-4 illustrated in Fig. 1A.

### DESCRIPTION OF EMBODIMENTS

In the following description, an embodiment of the present invention will be described with reference to the drawings. In the drawings that will be described below, identical or corresponding components will be indicated by the same reference numerals, and redundant descriptions will be omitted.

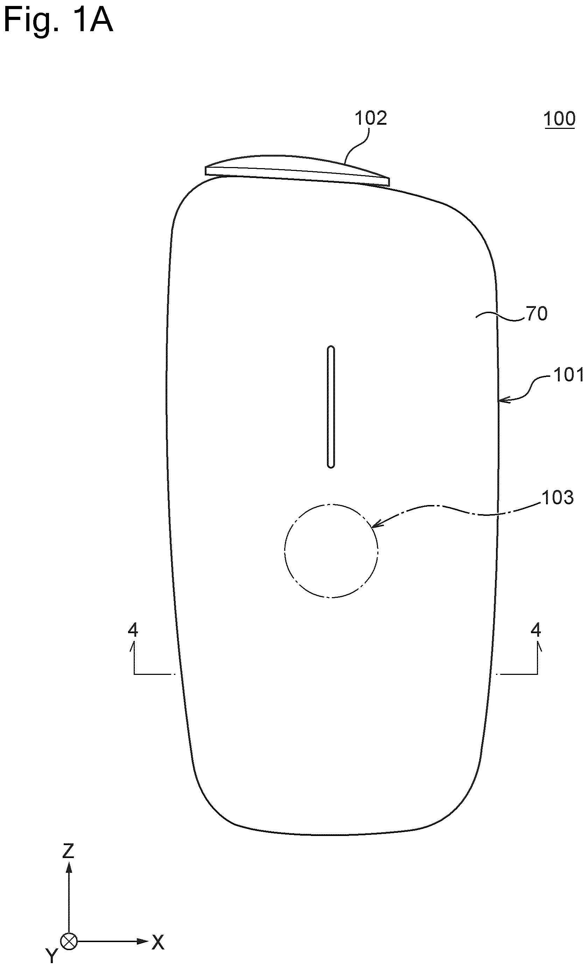

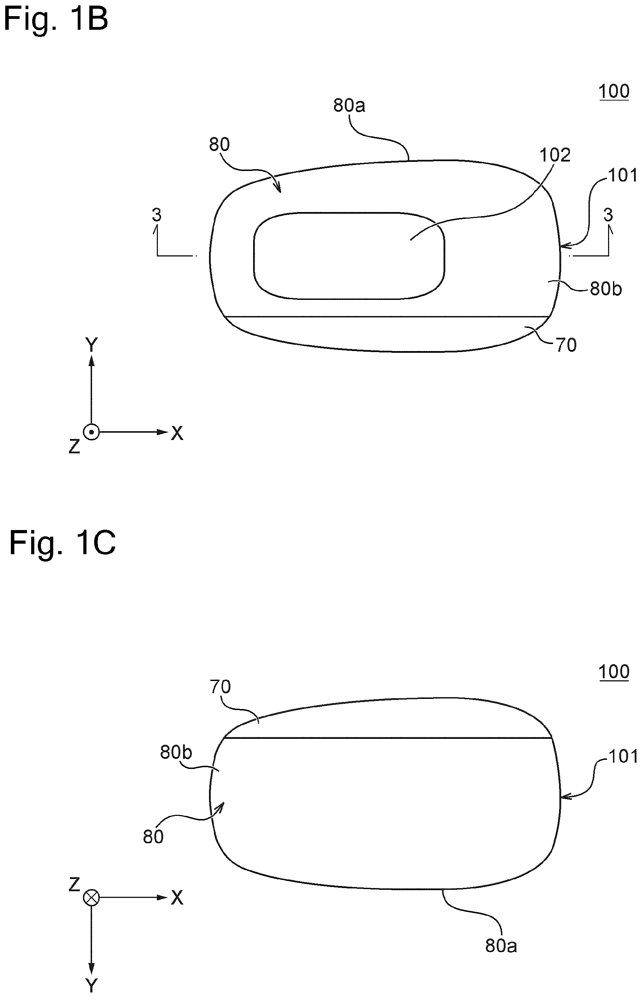

Fig. 1A is a schematic front view of a flavor inhaler 100 according to the present embodiment. Fig. 1B is a schematic top view of the flavor inhaler 100 according to the present embodiment. Fig. 1C is a schematic bottom view of the flavor inhaler 100 according to the present embodiment. In the drawings that will be described in the present specification, an X-Y-Z orthogonal coordinate system may be set for convenience of the description. In this coordinate system, a Z axis extends vertically upward. An X-Y plane is laid so as to cut across the flavor inhaler 100 horizontally. A Y axis is disposed so as to extend from the front side to the back side of the flavor inhaler 100. The Z axis can also be said to be an insertion direction of a consumable contained in a chamber 50 of an atomization unit 30, which will be described below. Further, the X-axis direction can also be said to be a device longitudinal direction in a plane perpendicular to the insertion direction of the consumable, or a direction in which a heating unit and a power source unit are lined up. The Y-axis direction can also be said to be a device lateral direction in the plane perpendicular to the insertion direction of the consumable.

The flavor inhaler 100 according to the present embodiment is configured to generate an aerosol by heating a smokable substance in the consumable. More specifically, the flavor inhaler 100 is configured to generate an aerosol that contains a flavor by heating, for example, a stick-type consumable having a flavor source including an aerosol source.

As illustrated in Figs. 1A to 1C, the flavor inhaler 100 includes an outer housing 101 (corresponding to one example of a housing), a slide cover 102, and a switch unit 103. The outer housing 101 constitutes the outermost housing of the flavor inhaler 100, and is sized so as to be contained inside a user's hand. When the user uses the flavor inhaler 100, the user can inhale the aerosol while holding the outer housing 101 with his/her hand.

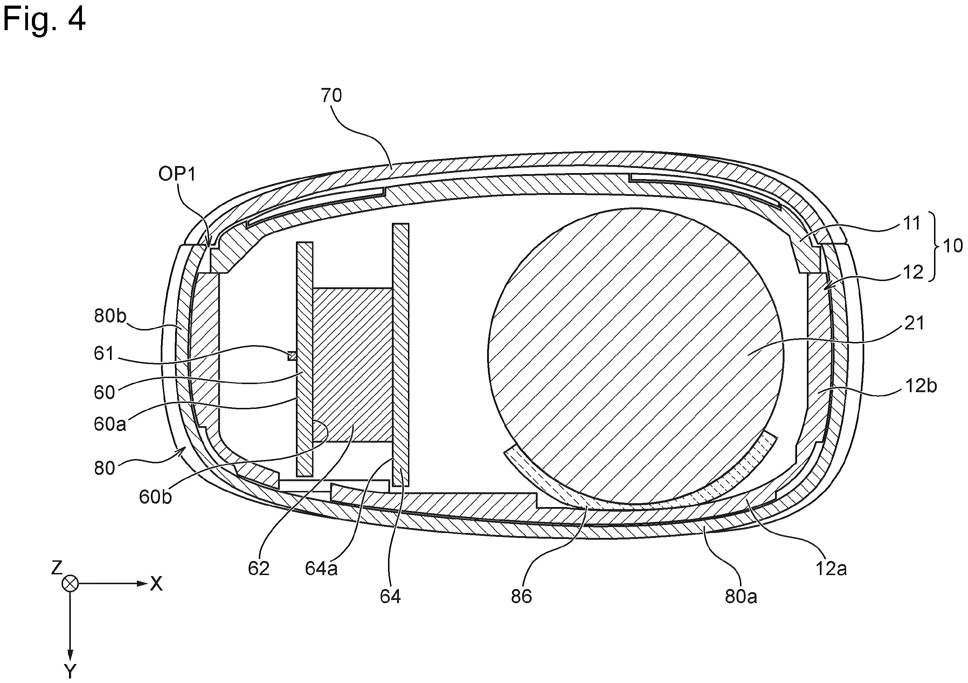

As illustrated in Figs. 1B and 1C, the outer housing 101 includes an outer panel 70 (corresponding to one example of a second housing) and an outer case 80 (corresponding to one example of a first housing) so as to be divided in the Y-axis direction. In other words, the outer housing 101 can be constructed by connecting the outer panel 70 to the outer case 80 in the Y-axis direction. The outer panel 70 can be configured to be detachably attachable to the outer case 80. The outer case 80 includes a bottom wall 80a, a circumferential wall 80b extending from the bottom wall 80a, and an opening OP1 defined by the circumferential wall 80b (refer to Fig. 4, which will be described below). The outer panel 70 is configured to close the opening OP1 (refer to Fig. 4, which will be described below) of the outer case 80.

The slide cover 102 is slidably attached to the outer housing 101 so as to close a not-illustrated opening portion for inserting the consumable. More specifically, the slide cover 102 is configured movably along the outer surface of the outer housing 101 between a closing position (the position illustrated in Figs. 1A and 1B), at which the slide cover 102 closes the not-illustrated opening portion of the outer housing 101, and an opening position, at which the slide cover 102 opens the not-illustrated opening portion. For example, the user can move the slide cover 102 to the closing position and the opening position by operating the slide cover 102 manually. Due to that, the slide cover 102 can permit or restrict access of the consumable to inside the flavor inhaler 100.

The switch unit 103 is used to switch on and off the actuation of the flavor inhaler 100. For example, the user can cause power to be supplied from a not-illustrated battery to the not-illustrated heating unit and heat the consumable without burning it by operating the switch unit 103 in a state that the consumable is inserted in the flavor inhaler 100. The switch unit 103 may include a switch provided outside the outer housing 101 or may include a switch located inside the outer housing 101. In the case where the switch is located inside the outer housing 101, the switch is indirectly pressed by pressing of the switch unit 103 on the surface of the outer housing 101. The present embodiment will be described citing the example in which the switch of the switch unit 103 is located inside the outer housing 101.

The flavor inhaler 100 may further include a not-illustrated terminal. The terminal can be an interface that connects the flavor inhaler 100 to, for example, an external power source. In a case where the power source provided to the flavor inhaler 100 is a chargeable battery, connecting the external power source to the terminal allows a current to be supplied from the external power source to the battery and the battery to be charged. Further, the flavor inhaler 100 may be configured in such a manner that data relating to the actuation of the flavor inhaler 100 can be transmitted to an external apparatus by connecting a data transmission cable to the terminal.

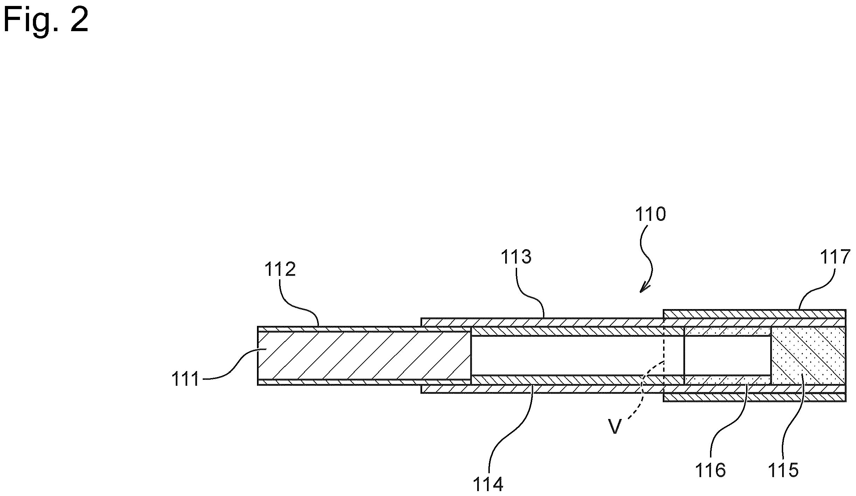

Next, the consumable used in the flavor inhaler 100 according to the present embodiment will be described. Fig. 2 is a schematic side cross-sectional view of a consumable 110. In the present embodiment, a smoking system can be constituted by the flavor inhaler 100 and the consumable 110. In the example illustrated in Fig. 2, the consumable 110 includes a smokable substance 111, a tubular member 114, a hollow filter unit 116, and a filter unit 115. The smokable substance 111 is wrapped with first rolling paper 112. The tubular member 114, the hollow filter unit 116, and the filter unit 115 are wrapped with second rolling paper 113 different from the first rolling paper 112. The second rolling paper 113 is also wrapped around a part of the first rolling paper 112 wrapped around the smokable substance 111. Due to that, the tubular member 114, the hollow filter unit 116, and the filter unit 115, and the smokable substance 111 are joined with each other. However, the second rolling paper 113 may be omitted, and the tubular member 114, the hollow filter unit 116, and the filter unit 115, and the smokable substance 111 may be joined with each other using the first rolling paper 112. A lip release agent 117, which is used to make it difficult for the user's lip to stick to the second rolling paper 113, is applied to the outer surface near the end portion of the second rolling paper 113 on the filter unit 115 side. A portion of the consumable 110 to which the lip release agent 117 is applied functions as a mouthpiece of the consumable 110. Further, an opening hole V may be concentrically provided in the circumferential direction of the tubular member 114, which fulfills a cooling segment function in the consumable 110. The opening hole V provided to the tubular member 114 is normally a hole for promoting an inflow of air from outside according to the user's inhalation, and the temperatures of a component and air flowing in from the smokable substance 111 can be lowered with the aid of this inflow of the air.

The smokable substance 111 can include the flavor source such as tobacco, and the aerosol source. Further, the first rolling paper 112 wrapped around the smokable substance 111 can be a breathable sheet member. The tubular member 114 can be a paper tube or a hollow filter. The consumable 110 includes the smokable substance 111, the tubular member 114, the hollow filter unit 116, and the filter unit 115 in the illustrated example, but the configuration of the consumable 110 is not limited thereto. For example, the hollow filter unit 116 may be omitted, and the tubular member 114 and the filter unit 115 may be disposed adjacent to each other. Further, although being not especially limited, airflow resistance in the longitudinal direction per consumable 110 is normally 8 mmH₂O or higher, preferably 10 mmH₂O or higher, and further preferably 12 mmH₂O or higher, and is normally 100 mmH₂O or lower, preferably 80 mmH₂O or lower, and further preferably 60 mmH₂O or lower from a viewpoint of ease of smoking.

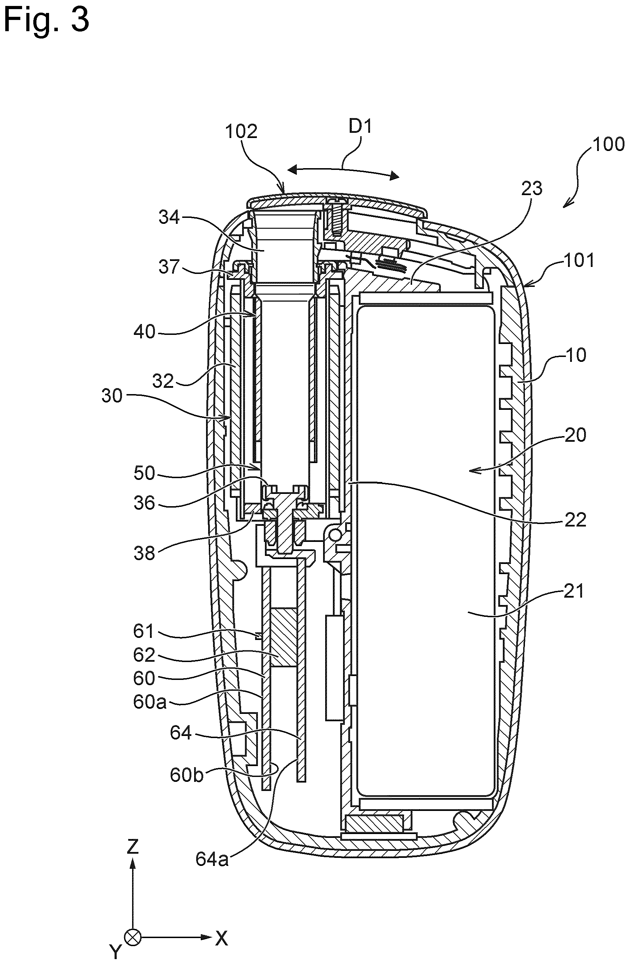

Next, the inner structure of the flavor inhaler 100 will be described. Fig. 3 is a schematic cross-sectional view of the flavor inhaler 100 as viewed from arrows 3-3 illustrated in Fig. 1B. Fig. 4 is a schematic cross-sectional view of the flavor inhaler 100 as viewed from arrows 4-4 illustrated in Fig. 1A. As illustrated in Figs. 3 and 4, an inner panel 10 is contained inside the outer housing 101 of the flavor inhaler 100. The inner panel 10 is made from, for example, resin, and, especially, can be made from polycarbonate (PC), ABS (Acrylonitrile-Butadiene-Styrene) resin, PEEK (polyetheretherketone), a polymer alloy containing a plurality of kinds of polymers, or the like, or metal such as aluminum. The inner panel 10 is preferably made from PEEK from viewpoints of thermal resistance and strength. However, the material of the inner panel 10 is not especially limited. The inner panel 10 forms the housing of the flavor inhaler 100 together with the outer housing 101, and a power source unit 20, an atomization unit 30 for the consumable 110, a first board 60, and a second board 64 (corresponding to one example of a component) are contained in an inner space of this housing. As illustrated in Fig. 4, the inner panel 10 includes a first inner panel 11 (corresponding to one example of the second housing) and a second inner panel 12 (corresponding to one example of the first housing). The inner panel 10 can be opened and closed by detaching and attaching the first inner panel 11 from and to the second inner panel 12. Further, the outer housing 101 is made from, for example, resin, and, especially, can be made from polycarbonate (PC), ABS (Acrylonitrile-Butadiene-Styrene) resin, PEEK (polyetheretherketone), a polymer alloy containing a plurality of kinds of polymers, or the like, or metal such as aluminum.

The power source unit 20 includes a battery 21. The battery 21 can be, for example, a chargeable battery. The battery 21 is electrically connected to a heating unit 40 of the atomization unit 30 via the first board 60. Due to that, the battery 21 can supply power to the heating unit 40 so as to appropriately heat the consumable 110.

As illustrated in Fig. 3, the atomization unit 30 includes the chamber 50 extending in the insertion direction of the consumable 110 (the Z-axis direction), the heating unit 40 surrounding a part of the chamber 50, a heat insulation unit 32, and a substantially tubular insertion guide member 34. The chamber 50 is configured to contain the consumable 110. The chamber 50 is preferably made from a material high in thermal resistance and low in thermal expansion coefficient, and can be made from, for example, metal such as stainless steel, resin such as PEEK, glass, or ceramic. As illustrated, a bottom member 36 may be provided on the bottom portion of the chamber 50. The bottom member 36 can function as a stopper that positions the consumable 110 inserted in the chamber 50. The bottom member 36 has a recess/protrusion on a surface with which the consumable 110 is in abutment, and can define a space usable to supply air to the surface with which the consumable 110 is in abutment. The bottom member 36 can be made from, for example, a resin material such as PEEK, metal, glass, or ceramic, but is not especially limited thereto. Further, the material for making the bottom member 36 may be a low thermally conductive member compared with the material for making the chamber 50. In a case where the bottom member 36 is joined with the bottom portion of the chamber 50, an adhesive that can be made from a resin material such as epoxy resin or an inorganic material can be used therefor.

The heating unit 40 is configured to heat the smokable substance 111 in the consumable 110 contained in the chamber 50 in contact with the outer peripheral surface of the chamber 50. More specifically, the heating unit 40 includes a heating element such as a heating track and an electric insulation sheet covering at least one surface of the heating element.

The heat insulation unit 32 is generally substantially tubular, and is disposed so as to surround the chamber 50. The heat insulation unit 32 can include, for example, an aerogel sheet. The insertion guide member 34 is made from a resin material such as PEEK, PC, or ABS, and is provided between the slide cover 102 located at the closing position and the chamber 50. Further, the flavor inhaler 100 includes a first holding unit 37 and a second holding unit 38 for holding the heat insulation unit 32. The first holding unit 37 and the second holding unit 38 can be made from, for example, elastomer such as silicone rubber. As illustrated in Fig. 3, the first holding unit 37 holds the end portion of the heat insulation unit 32 on the Z-axis positive direction side. Further, the second holding unit 38 holds the end portion of the heat insulation unit 32 on the Z-axis negative direction side.

The insertion guide member 34 has a function of guiding the insertion of the consumable 110. More specifically, when the slide cover 102 is located at the opening position, the insertion guide member 34 guides insertion of the consumable 110 into the chamber 50 in reaction to an attempt to insert the consumable 110 into the insertion guide member 34. In the present embodiment, the insertion guide member 34 can contact the chamber 50, and therefore the insertion guide member 34 is preferably made from PEEK from a viewpoint of thermal resistance. As illustrated in Fig. 3, the slide cover 102 is movable between the opening position and the closing position in a direction D1.

The flavor inhaler 100 includes a first chassis 22 extending in the Z-axis direction between the battery 21 and the atomization unit 30, and a second chassis 23 extending so as to cover the slide cover 102 side of the battery 21. The first chassis 22 and the second chassis 23 are configured to define a space where the battery 21 is contained in the inner panel 10.

As illustrated in Figs. 3 and 4, the first board 60 includes an electronic element 61. The electronic element 61 can be any element that can generate heat under a predetermined condition. The first board 60 includes a first surface 60a and a second surface 60b opposite from the first surface 60a and located at a longer distance from the housing (the outer housing 101 and the inner panel 10) than the distance between the first surface 60a and the housing. As will be used herein, the distance between the first board 60 and the housing refers to a distance in a direction perpendicular to the first surface 60a or the second surface 60b. Further, the second board 64 faces the second surface 60b of the first board 60. The first board 60 and the second board 64 can be, for example, PCBs.

In the present embodiment, a first heat dissipation member 62 is provided. When the electronic element 61 of the first board 60 generates heat, the first heat dissipation member 62 is used to dissipate this heat. Then, as illustrated in Fig. 4, the outer case 80 includes the opening OP1 defined by the circumferential wall 80b in addition to the bottom wall 80a and the circumferential wall 80b in the present embodiment. Further, the second inner panel 12 also includes a bottom wall 12a and a circumferential wall 12b extending from the bottom wall 12a, and the circumferential wall 12b defines a part of the opening OP1. The first board 60 is placed into the second inner panel 12 and the outer case 80 via the opening OP1, and therefore this direction in which the first board 60 is placed (the Y-axis direction in the present example) is substantially in parallel with the first surface 60a and the second surface 60b. In other words, the first surface 60a and the second surface 60b of the first board 60 are substantially in parallel with the direction in which the opening OP1 faces (the Y-axis direction in the present example). Therefore, if ever the first heat dissipation member 62 is disposed on the first surface 60a of the first board 60, the first heat dissipation member 62 may interfere with the circumferential wall 12b of the second inner panel 12 to hinder the assembling the flavor inhaler 100 when the first board 60 is placed into the second inner panel 12 and the outer case 80.

On the other hand, in the present embodiment, the first heat dissipation member 62 is interposed between a portion of the second surface 60b of the first board 60 that corresponds to the electronic element 61, and the second board 64 as illustrated in Figs. 3 and 4. Due to that, the heat of the electronic element 61 of the first board 60 can be transferred (dissipated) to the second board 64 facing the second surface 60b of the first board 60 via the first heat dissipation member 62, and therefore local heat generated in the first board 60 can be transferred to the second board 64 while being dispersed. In addition thereto, since the first heat dissipation member 62 is interposed between the second board 64 and the first board 60, the first heat dissipation member 62 can be prevented from contacting the housing to hinder the assembling of the flavor inhaler 100 when the first board 60, the first heat dissipation member 62, and the second board 64 are placed into the housing. In the present specification, the "portion of the second surface 60b of the first board 60 that corresponds to the electronic element 61" refers to a portion of the second surface 60b opposite from the electronic element 61 in a case where the electronic element 61 is mounted on the first surface 60a, or a portion of the second surface 60b on which the electronic element 61 is located in a case where the electronic element 61 is mounted on the second surface 60b.

Preferably, the electronic element 61 is a charging IC for controlling the charging of the battery 21. In this case, the heat can be dissipated from the charging IC to the second board 64 with the aid of the first heat dissipation member 62, and therefore an increase in the temperature of the charging IC can be suppressed even when a current of a higher value is supplied to the battery 21 to reduce the time taken to charge the battery 21 compared with a conventionally spent time.

Further, preferably, the second board 64 includes a surface 64a facing the second surface 60b of the first board 60 as illustrated in Figs. 3 and 4. Interposing the first heat dissipation member 62 between the second surface 60b of the first board 60 and the surface 64a of the second board 64 allows the local heat generated in the first board 60 to be transferred to the surface 64a of the second board 64 while being dispersed, thereby contributing to suppressing a local increase in the temperature of the second board 64.

Preferably, the first heat dissipation member 62 is a heat dissipation pad containing silicone. Such a heat dissipation pad containing silicone can efficiently transfer (dissipate) heat by being disposed in contact with a heat dissipation target so as to be compressed. Therefore, the first heat dissipation member 62 is preferably compressed by the second surface 60b of the first board 60 and the second board 64. Due to that, the heat can be efficiently dissipated from the first board 60 to the second board 64 via the first heat dissipation member 62. Preferably, the first heat dissipation member 62 is flexible. Due to that, the first heat dissipation member can adhere in close contact with the heat dissipation target, thereby working to efficiently dissipate the heat from the heat dissipation target. Further, the first heat dissipation member 62 is preferably elastic. Due to that, the first heat dissipation member 62 can be appropriately compressed by the second surface 60b of the first board 60 and the second board 64, thereby working to efficiently dissipate the heat from the heat dissipation target.

As illustrated in Figs. 3 and 4, the electronic element 61 is preferably mounted on the first surface 60a of the first board 60. In this case, the heat is dispersed in an in-plane direction in the first board 60 when the heat of the electronic element 61 is transferred from the first surface 60a to the second surface 60b of the first board 60, and therefore the locally generated heat of the electronic element 61 can be transferred to the first heat dissipation member 62 while being dispersed by the first board 60.

If a current of a higher value is supplied to the battery 21 to reduce the time taken to charge the battery 21, the battery 21 also generates heat to have a higher temperature than a conventionally reached temperature. Therefore, the flavor inhaler 100 preferably includes a second heat dissipation member 66 interposed between the surface of the battery 21 and the inner surface of the housing (the second inner panel 12) as illustrated in Fig. 4. Due to that, the heat of the battery 21 can be transferred (dissipated) to the housing via the second heat dissipation member 66. The battery 21 generates the heat not locally but entirely, and therefore it is preferable to dissipate the heat thereof to the housing having a relatively great heat capacity.

More specifically, the second heat dissipation member 66 is preferably interposed between the surface of the battery 21 and the inner surface of the bottom wall 12a of the second inner panel 12. In this regard, the second heat dissipation member 66 can be interposed between the surface of the battery 21 and the inner surface of the bottom wall 12a by placing the battery 21 into the outer case 80 via the opening OP1. Therefore, the second heat dissipation member 66 can be prevented from hindering the assembling of the flavor inhaler 100. An arbitrary member may be disposed between the bottom wall 12a and the second heat dissipation member 66.

Preferably, the second heat dissipation member 66 is, for example, a heat dissipation pad containing silicone, similarly to the first heat dissipation member 62. Preferably, the second heat dissipation member 66 is compressed by the surface of the battery 21 and the inner surface of the housing (the second inner panel 12), similarly to the first heat dissipation member 62. Due to that, the heat can be efficiently dissipated from the battery 21 to the housing via the second heat dissipation member 66. Preferably, the second heat dissipation member 66 is flexible. Due to that, the second heat dissipation member 66 can adhere in close contact with the heat dissipation target (the battery 21) as illustrated in Fig. 4, thereby working to efficiently dissipate the heat from the heat dissipation target. Further, the second heat dissipation member 66 is preferably elastic. Due to that, the second heat dissipation member 66 can be appropriately compressed by the surface of the battery 21 and the inner surface of the housing (the second inner panel 12), thereby working to efficiently dissipate the heat from the heat dissipation target.

The present embodiment has been described assuming that the flavor inhaler 100 includes the inner panel 10, but the flavor inhaler 100 does not have to include the inner panel 10. In this case, the second heat dissipation member 66 can be interposed between the surface of the battery 21 and the inner surface of the bottom wall 80a of the outer case 80.

Having described the embodiment of the present invention, the present invention shall not be limited to the above-described embodiment, and can be modified in various manners within the scope of the technical idea disclosed in the claims, specification, and drawings. Note that any shape and material not directly described or illustrated in the specification or drawings are still within the scope of the technical idea of the present invention insofar as they allow the present invention to achieve the actions and effects thereof. For example, the heating unit 40 is not limited to the resistively heating-type heating unit and may be an inductively heating-type heating unit. In this case, the heating unit 40 can heat the chamber 50 by induction heating. Further, in a case where the consumable 110 includes a susceptor, the heating unit 40 can heat the susceptor of the consumable 110 by induction heating. Further, the heating unit 40 may include an internally heating-type heater that heats the consumable 110 from inside. The consumable 110 may be a tank that contains liquid. In this case, the heating unit 40 can be configured to atomize the liquid in the tank.

### REFERENCE SIGNS LIST

- 10: inner panel
- 11: first inner panel
- 12: second inner panel
- 12a: bottom wall
- 12b: circumferential wall
- 21: battery
- 60: first board
- 60a: first surface
- 60b: second surface
- 61: electronic element
- 62: first heat dissipation member
- 64: second board
- 64a: surface
- 66: second heat dissipation member
- 70: outer panel
- 80: outer case
- 80a: bottom wall
- 80b: circumferential wall
- 100: flavor inhaler
- 101: outer housing
- 111: smokable substance
- OP1: opening

## Claims

1. A flavor inhaler configured to generate an aerosol by heating a smokable substance, the flavor inhaler comprising:
a first board including an electronic element; and
a housing containing the first board,
the first board including a first surface and a second surface, the second surface being opposite from the first surface and located at a longer distance from the housing than a distance between the first surface and the housing,
the flavor inhaler further comprising:
a component facing the second surface of the first board; and
a first heat dissipation member interposed between a portion of the second surface of the first board that corresponds to the electronic element and the component.

2. The flavor inhaler according to claim 1, wherein the electronic element is a charging IC for controlling charging of a battery.

3. The flavor inhaler according to claim 1 or 2, wherein the first heat dissipation member is a heat dissipation pad containing silicone.

4. The flavor inhaler according to claim 3, wherein the first heat dissipation member is compressed by the second surface of the first board and the component.

5. The flavor inhaler according to any one of claims 1 to 4, wherein the component is a second board having a surface facing the second surface of the first board.

6. The flavor inhaler according to any one of claims 1 to 5, wherein the electronic element is mounted on the first surface of the first board.

7. The flavor inhaler according to any one of claims 1 to 6, further comprising:
a battery; and
a second heat dissipation member interposed between a surface of the battery and an inner surface of the housing.

8. The flavor inhaler according to claim 7, wherein the housing includes a first housing including a bottom wall, a circumferential wall, and an opening defined by the circumferential wall, and a second housing configured to close the opening, and
wherein the second heat dissipation member is interposed between the surface of the battery and an inner surface of the bottom wall.
